# EUROPÄISCHE PATENTSCHRIFT

(11) **EP 4 223 325 B1**
(45) Veröffentlichungstag und Bekanntmachung des Hinweises auf die Patenterteilung: **16.07.2025**
(21) Anmeldenummer: 23155142.5
(22) Anmeldetag: 06.02.2023
(51) Int. Cl.: A61L 27/18, A61L 27/34, A61L 27/50

(54) **OBERFLÄCHENBEHANDLUNG VON POLYARYLETHERKETONEN**
SURFACE TREATMENT OF POLYARYLETHERKETONES
TRAITEMENT DE SURFACE DE POLYARYLÉTHERCÉTONES

(30) Priorität: 08.02.2022 DE 102022102870
(43) Veröffentlichungstag der Anmeldung: 09.08.2023
(73) Patentinhaber: Fraunhofer-Gesellschaft zur Förderung der angewandten Forschung e.V., 80686 München (DE)
(72) Erfinder: SCHMELZER, Christian, 06120 Halle (Saale) (DE); FRIEDMANN, Andrea, 06120 Halle (Saale) (DE); RUBERS, Julius, 06120 Halle (Saale) (DE); KÜRBITZ, Tobias, 06366 Köthen (Anhalt) (DE); HEDTKE, Tobias, 06120 Halle (Saale) (DE); HERBST, Christian, 06120 Halle (Saale) (DE)
(74) Vertreter: Maiwald GmbH

(56) Entgegenhaltungen:
- DE-A1- 102015 002 398
- MA ZHANGYU ET AL: "Biologically Modified Polyether Ether Ketone as Dental Implant Material", FRONTIERS IN BIOENGINEERING AND BIOTECHNOLOGY, vol. 8, 18 December 2020 (2020-12-18), pages 1 - 17, XP093026692, DOI: 10.3389/fbioe.2020.620537

## Beschreibung

Die vorliegende Erfindung betrifft ein Verfahren zur Oberflächenbehandlung von Polyaryletherketonen mit Aldehyden, ein Verfahren zur Funktionalisierung von oberflächenbehandelten Polyaryletherketonen und einen Gegenstand, der eine funktionalisiertes Polyaryletherketon umfasst.

### Technischer Hintergrund

Polyetheretherketon (PEEK) ist ein thermoplastisches Hochleistungspolymer, das aufgrund seiner mechanischen Eigenschaften für Implantate in der Wirbelsäulen-, Gesichts- und Unfallchirurgie verwendet wird. Das Material ist seit etwa 20 Jahren für die medizinische Verwendung zugelassen, doch seine breite Anwendung hat sich verzögert. Grund dafür sind die Oberflächeneigenschaften von PEEK, die die Integration des Implantats in dem umliegenden Gewebe schwierig oder sogar unmöglich machen.

Aufgrund der Kombination aus hervorragender thermischer Stabilität, mechanischen Eigenschaften sowie einfacher Verarbeitung wird das polyaromatische, teilkristalline, thermoplastische Polymer PEEK als Material für Hochleistungsanwendungen eingesetzt. Es besteht aus einer Molekülkette aus einem aromatischen Grundgerüst, das durch funktionelle Keton- und Ethergruppen verbunden ist. Die chemische Struktur der polyaromatischen Ketone ermöglicht Stabilität bei hohen Temperaturen über 300 °C, Beständigkeit gegen chemische und Strahlenschäden sowie Kompatibilität mit vielen verstärkenden Materialien wie Glas- und Kohlenstofffasern.

Der Hauptvorteil von medizinischem PEEK als Implantatmaterial gegenüber Implantaten aus Standardmetalllegierungen ist das reduzierte Elastizitätsmodul (3 - 4 GPa), der dem der menschlichen Kortikalis nahekommt. Darüber hinaus ist PEEK röntgen- und MRTkompatibel.

Nach der Implantation wird das Wasser innerhalb von Nanosekunden an der Implantatoberfläche adsorbiert. Die Orientierung der polaren Wassermoleküle auf der PEEK-Oberfläche wird durch die Oberflächeneigenschaften des Polymers beeinflusst. Da das Implantat mit Blutplasma in Kontakt kommt (das mehr als 5000 Proteine enthält), sind die nachfolgenden Interaktionen der Proteine mit der Polymeroberfläche folglich von der Ausrichtung dieser ursprünglich adsorbierten Wassermoleküle beeinflusst. Die Wechselwirkungen dieser Proteine mit der Polymeroberfläche werden durch die Oberflächenchemie, Oberflächenladung und Oberflächenstruktur beeinflusst, die über den Erfolg oder Misserfolg eines Implantats in einer bestimmten Anwendung bestimmen. Im Allgemeinen zeichnet sich PEEK durch eine bioinerte Oberflächenchemie mit hydrophoben Eigenschaften aus. Diese Oberflächeneigenschaften behindern die Zellanhaftung erheblich, was zu einer schlechten Implantatintegration und folglich zu einem Implantatversagen führt. Die biologische Reaktion auf der PEEK-Oberfläche kann eine Fremdkörperreaktion hervorrufen, die wiederum zu einer Lockerung des Implantats bei der Verkapselung des Implantats führen kann.

Um die Osseointegration von PEEK-Implantaten zu verbessern, sollten biologische Wechselwirkungen mit Komponenten der extrazellulären Matrix ermöglicht werden. Nach dem derzeitigen Stand der Technik wurde dies in verschiedenen Ansätzen durch Modifizierung der Materialoberfläche versucht. So werden beispielsweise eine Oberflächenfunktionalisierung durch Plasmabehandlungen (z. B. mit O₂, N₂) sowie durch Beschichtungsverfahren, die auf eine metallische Beschichtung (z. B. Ti, TiO₂, Ta), keramische Beschichtung (z. B. Zeolith, Hydroxylapatit) oder biologische Beschichtung (BMP, morphogene Knochenproteine) abzielen, verwendet. Die plasmabasierte Oberflächenfunktionalisierung von PEEK führt allerdings lediglich zu minimalen und kurzfristig stabilen Wirkungen im Hinblick auf biologische Wechselwirkungen.

DE102015002398 A1 offenbart ein Material für Knochenimplantate, umfassend: (a) eine Oberfläche, umfassend ein Material, ausgewählt aus der Gruppe, bestehend aus oxidischen Keramikmaterialien, Titan, Polymermaterialien und Kompositmaterialien, (b) eine kovalent an diese Oberfläche gebundene Matrix, umfassend Kollagen und/oder Gelatine, und (c) in diese Matrix eingelagertes Calciumphosphat. In einer Ausführungsform, die Oberfläche umfasst PEEK und das Kollagen und/oder die Gelatine ist an das PEEK über einen Linker, ausgewählt aus der Gruppe, bestehend aus einem Dicarbonsäure-Linker, einem Maleimid-Linker und einem Hexamethylendiisocyanat-Linker gebunden.

Aufgrund der guten Osseointegration von aufgerauten Titanoberflächen ist eine gängige Methode zur Verbesserung der Osseointegration von PEEK die Beschichtung von PEEK mit aufgerautem Titan. Metallische Beschichtungen von PEEK-Implantatoberflächen können zwar zu einer besseren Osseointegration beitragen, bringen jedoch auch Nachteile mit sich. Einerseits verursacht eine solche Beschichtung eine erhebliche Veränderung in Bezug auf Verarbeitung, Zulassung und Anwendung des Implantats als Verbundwerkstoff; zum anderen wird der Vorteil von PEEK in Bezug auf die radiologische Diagnostik beeinträchtigt. Außerdem wurde der vorzeitige Verschleiß solcher Beschichtungen beschrieben.

Um biologische Beschichtungen (Seidenfibroin, BMP) auf bioinerten PEEK-Oberflächen realisieren zu können, werden diese z.B. mit Schwefelsäure geätzt, wodurch die PEEK-Oberfläche aufgeraut wird.

Eine Funktionalisierung von PEEK-Oberflächen im Hinblick auf biologische Wechselwirkungen, um beispielsweise die Bindung an Protein deutlich zu verbessern ohne die Oberflächenstruktur des PEEK zu beeinträchtigen, ist - wie an den drei aufgeführten Beispielen gezeigt - im Stand der Technik nicht bekannt.

Die vorliegende Erfindung beschreibt eine Methode der Vorbehandlung von Polyaryletherketonen (PAEKs), vorzugsweise PEEK, mit Aldehyden. Die hier beschriebene Vorbehandlung von PAEK-Oberflächen ermöglicht die Funktionalisierung durch kovalente chemische Bindungen. Diese Methode kann insbesondere eingesetzt werden zur Vorbehandlung vor einer Beschichtung von PAEKs mit Biomolekülen, ist j edoch nicht darauf beschränkt.

### Zusammenfassung der Erfindung

Die vorliegende Erfindung betrifft ein Verfahren zur Oberflächenbehandlung von Polyaryletherketonen (PAEKs) umfassend folgende Schritte:
- Bereitstellen eines Gegenstandes, der ein oder mehrere Polyaryletherketone (PAEKs) enthält;
- in Kontakt bringen von mindestens einer Teilfläche der Oberfläche des Gegenstandes, der ein oder mehrere Polyaryletherketone (PAEKs) enthält, mit einem Aldehyd,
wobei das Aldehyd mit dem/den Polyaryletherketon(en) (PAEKs) an der mindestens einen Teilfläche der Oberfläche des Gegenstands unter Ausbildung einer Hydroxyalkyl- und/oder Hydroxyarylgruppe reagiert.

Weiterhin betrifft die vorliegende Erfindung ein Verfahren zur Funktionalisierung von oberflächenbehandelten Polyaryletherketonen (PAEKs), umfassend folgende Schritte:
a) Behandeln von mindestens einer Teilfläche der Oberfläche eines Gegenstandes, der ein oder mehrere Polyaryletherketone (PAEKs) enthält mit dem hierin beschriebenen Verfahren zur Oberflächenbehandlung von Polyaryletherketonen (PAEKs);
b) Beschichten der mindestens einen behandelten Teilfläche der Oberfläche des Gegenstandes mit einer Zusammensetzung, die eine chemische Verbindung enthält mit chemischen Gruppen, die eine kovalente Bindung mit den auf der Oberfläche des Gegenstandes ausgebildeten Hydroxyalkyl- und/oder Hydroxyarylgruppen ausbilden können.

Des Weiteren betrifft die vorliegende Erfindung einen Gegenstand enthaltend ein oder mehrere Polyaryletherketone (PAEKs) und eine Beschichtung auf mindestens einer Teilfläche der Oberfläche des Gegenstands, wobei an der mindestens einen beschichteten Teilfläche der Oberfläche des Gegenstands der/die Polyaryletherketone (PAEKs) Hydroxyalkyl- und/oder Hydroxyarylgruppen enthält/enthalten; und
mindestens ein Teil der Hydroxyalkyl- und/oder Hydroxyarylgruppen des Polyaryletherketons/der Polyaryletherketone (PAEKs) kovalente Bindungen mit chemischen Gruppen von mindestens einer chemischen Verbindung in der Beschichtung ausgebildet hat.

### Ausführliche Beschreibung der Erfindung

### Verfahren zur Oberflächenbehandlung von Polyaryletherketonen (PAEK)

In einem ersten Aspekt betrifft die vorliegende Erfindung ein Verfahren zur Oberflächenbehandlung von Polyaryletherketonen (PAEK) umfassend folgende Schritte:
- Bereitstellen eines Gegenstandes, der ein oder mehrere Polyaryletherketone (PAEKs) enthält;
- in Kontakt bringen von mindestens einer Teilfläche der Oberfläche des Gegenstandes, der ein oder mehrere Polyaryletherketone (PAEKs) enthält, mit einem Aldehyd,
wobei das Aldehyd mit dem/den Polyaryletherketon(en) (PAEKs) an der mindestens einen Teilfläche der Oberfläche des Gegenstands unter Ausbildung einer Hydroxyalkyl- und/oder Hydroxyarylgruppe reagiert.

Das Polymergerüst der Polyaryletherketone (PAEKs) besteht aus 1,4-substituierten Arylgruppen (R), die durch Keton- (R-CO-R) und/oder Ethergruppen (R-O-R) verbunden sind.

Die Polyaryletherketone (PAEKs) sind vorzugsweise ausgewählt aus Polyetherketonen (PEK), Polyetheretherketonen (PEEK), Polyetherketonketonen (PEKK), Polyetheretheretherketonen (PEEEK), Polyetheretherketonketonen (PEEKK), Polyetherketonetherketonketonen (PEKEKK) oder Mischungen daraus. Besonders bevorzugt sind Polyetheretherketone (PEEK).

Polyetheretherketon (PEEK) hat die allgemeine chemische Formel (-R-O-R-O-R-CO-)ₙ. Polyetherketon (PEK) hat die allgemeine chemische Formel (-O-R-CO-R-)ₙ. Polyetherketonketon (PEKK) hat die allgemeine chemische Formel (-R-O-R-CO-R-CO-)ₙ. Polyetheretheretherketon (PEEEK) hat die allgemeine chemische Formel (-R-O-R-O-R-O-R-CO-)ₙ.

Polyetheretherketonketon (PEEKK) hat die allgemeine chemische Formel (-R-O-R-O-R-CO-R-CO-)ₙ.

Polyetherketonetherketonketon (PEKEKK) hat die allgemeine chemische Formel (-R-O-R-CO-R-O-R-CO-R-CO-R-CO-)ₙ.

Dabei stellt R jeweils eine 1,4-substituierte Arylgruppe und n die Anzahl der Wiederholungen des jeweiligen Monomers dar.

Der Gegenstand kann einen oder mehrere Polyaryletherketone (PAEKs), wie beispielsweise einen Polyaryletherketon (PAEK) oder eine Mischung aus zwei bis fünf, wie beispielsweise zwei oder drei Polyaryletherketonen (PAEKs) enthalten. Vorzugsweise enthält der Gegenstand einen Polyaryletherketon (PAEK).

Der Gegenstand kann aus einem oder mehreren Polyaryletherketonen (PAEKs), wie beispielsweise einem Polyaryletherketon (PAEK) oder eine Mischung aus zwei bis fünf, wie beispielsweise zwei oder drei Polyaryletherketonen (PAEKs) bestehen. Vorzugsweise besteht der Gegenstand aus einem Polyaryletherketon (PAEK).

In einer anderen Ausführungsform enthält der Gegenstand eine Mischung aus einem oder mehreren Polyaryletherketonen (PAEKs) mit einem anorganischen Strukturmaterial, vorzugsweise mit Glasfasern, Kohlenstofffasern und/oder Hydroxyapatit. Dabei kann der Gegenstand einen oder mehrere Polyaryletherketone (PAEKs), wie beispielsweise einen Polyaryletherketon (PAEK) oder eine Mischung aus zwei bis fünf, wie beispielsweise zwei oder drei Polyaryletherketonen (PAEKs) enthalten. Vorzugsweise enthält der Gegenstand einen Polyaryletherketon (PAEK).

Der Gewichtsanteil an anorganischem Strukturmaterial liegt dabei bevorzugt im Bereich von 10 bis 50 Gewichts-%, stärker bevorzugt im Bereich von 20 bis 30 Gewichts-%, bezogen auf das Gesamtgewicht des Gegenstands.

Der Gewichtsanteil des/der Polyaryletherketon(e) (PAEKs) liegt bevorzugt in Bereich von 50 bis 90 Gewichts-%, stärker bevorzugt im Bereich von 70 bis 80 Gewichts-%, bezogen auf das Gesamtgewicht des Gegenstands.

Der Gegenstand kann jegliche Form aufweisen. Vorzugsweise ist der Gegenstand bereits in die Form gebracht, in der er nach der Behandlung mit dem/den erfindungsgemäßen Verfahren eingesetzt werden soll.

Wichtig ist, dass unabhängig von dem Gewichtsanteil der Polyaryletherketon(e) (PAEK) die zu behandelnde(n) Oberfläche(n) des Gegenstands Polyaryletherketongruppen aufweist, die mit Aldehyd reagieren können.

Mindestens eine Teilfläche der Oberfläche, vorzugsweise die ganze Oberfläche des Gegenstandes wird mit einem Aldehyd in Kontakt gebracht.

Das Aldehyd ist vorzugsweise ein linearer oder verzweigter aliphatischer oder aromatischer Kohlenwasserstoff, vorzugsweise ein linearer aliphatischer Kohlenwasserstoff, mit 1 bis 12, bevorzugt 1 bis 7, stärker bevorzugt 1 bis 5 Kohlenstoffatomen mit 1 bis 4, vorzugsweise 1 oder 2 Aldehydgruppen.

Das Aldehyd ist vorzugsweise ausgewählt aus Formaldehyd (Methanal), Glyoxal (Ethandial), Succinaldehyd (Butandial) und Glutaraldehyd (Pentandial) oder Mischungen daraus. Besonders bevorzugt ist Formaldehyd.

Das Aldehyd liegt vorzugsweise als Lösung, vorzugsweise als wässrige Lösung vor. Diese Lösung enthält üblicherweise das Aldehyd in einer Konzentration von 25 bis 75%, vorzugsweise 30 bis 55%.

Das Aldehyd kann mit der mindestens einen Teilfläche der Oberfläche des Gegenstands als Flüssigkeit, als Gas oder als Aerosol in Kontakt gebracht werden.

Üblicherweise erfolgt dieser Schritt über einen begrenzten Zeitraum, wie beispielsweise 10 min bis 60 min, vorzugsweise 15 min bis 45 min, besonders bevorzugt 30 min.

Dabei kann der Gegenstand in eine flüssige Aldehydlösung eingetaucht werden. Die Temperatur der Flüssigkeit spielt dabei eine untergeordnete Rolle und liegt vorzugsweise im Bereich von 10 bis 50°C, vorzugsweise 15 bis 30°C (üblicherweise abhängig vom Siedepunkt der Lösung).

Nach Ablauf der Kontaktzeit wird der Gegenstand vorzugsweise aus der flüssigen Aldehydlösung entfernt und die überschüssige Aldehydlösung von der mindestens einer Oberfläche entfernt, beispielsweise durch Abstreifen oder Abtupfen.

Bei in Kontakt bringen der mindestens einen Teilfläche der Oberfläche des Gegenstands mit dem Aldehyd in gasförmiger oder in Aerosolform wird der Gegenstand vorzugsweise in einem versiegelten Begasungsraum, wie einer Begasungskammer oder einem Begasungskasten platziert, in den das Aldehydgas oder -aerosol eingespeist wird.

Nach Ablauf der Kontaktzeit wird das Aldehydgas oder -aerosol aus dem Begasungsraum evaporiert und der Gegenstand aus dem Begasungsraum entfernt. Etwaiges überschüssiges Aldehyd wird vorzugsweise von der mindestens einen Teilfläche der Oberfläche entfernt, beispielsweise durch Abstreifen oder Abtupfen.

Durch den Kontakt der Polyaryletherketongruppen auf der mindestens einen Teilfläche der Oberfläche des Gegenstandes mit dem Aldehyd kommt es zu einer chemischen Reaktion des Aldehyds mit den Polyaryletherketongruppen, vorzugsweise mit Kohlenwasserstoffgruppen der Polyaryletherketongruppen, insbesondere mit Kohlenwasserstoffgruppen der Arylgruppen der Polyaryletherketongruppen auf der mindestens einen Teilfläche der Oberfläche des Gegenstandes. Hierbei wird vorzugsweise aus der Kohlenwasserstoffgruppe und der Aldehydgruppe eine Hydroxyalkyl- und/oder Hydroxyarylgruppe ausgebildet, vorzugsweise wird eine Methylolgruppe an die Kohlenwasserstoffgruppe addiert.

Diese so gebildeten Hydroxyalkyl- und/oder Hydroxyarylgruppen vorzugsweise die so addierten Methylolgruppen, auf der mindestens einen Teilfläche der Oberfläche des Gegenstandes, sind geeignet, in einem weiteren Verfahren der vorliegenden Erfindung eine kovalente Bindung mit geeigneten chemischen Gruppen einer Beschichtung einzugehen.

Die mindestens eine Teilfläche der Oberfläche des Gegenstandes, der ein oder mehrere Polyaryletherketone (PAEKs) enthält, kann zusätzlich mindestens einer weiteren Oberflächenbehandlung, wie beispielsweise einer Plasmabehandlung mit z.B. O₂ oder N₂, unterzogen werden. Eine solche Plasmabehandlung kann die Anzahl von reaktiven Bindungsstellen auf der mindestens einen Teilfläche der Oberfläche des Gegenstands für kovalente Bindungen mit geeigneten chemischen Gruppen einer Beschichtung erhöhen. Die Plasmabehandlung kann in einem Verfahrensschritt nach dem Verfahrensschritt des in-Kontakt-Bringens von mindestens einer Teilfläche der Oberfläche des Gegenstandes, der Polyaryletherketon (PAEK) enthält, mit einem Aldehyd durchgeführt werden.

Die Plasmabehandlung kann in einem Verfahrensschritt vor dem Verfahrensschritt des in-Kontakt-Bringens von mindestens einer Teilfläche der Oberfläche des Gegenstandes, der Polyaryletherketon (PAEK) enthält, mit einem Aldehyd durchgeführt werden. Hierdurch kann die Haftung der Beschichtung auf der mindestens einen Teilfläche der Oberfläche des Gegenstandes erhöht werden.

### Verfahren zur Funktionalisierung von oberflächenbehandelten Polyaryletherketonen (PAEKs)

In einem weiteren Aspekt betrifft die vorliegende Erfindung ein Verfahren zur Funktionalisierung von oberflächenbehandelten Polyaryletherketonen (PAEKs), umfassend folgende Schritte:
a) Behandeln von mindestens einer Teilfläche der Oberfläche eines Gegenstandes, der ein oder mehrere Polyaryletherketone (PAEKs) enthält, mit dem hierin beschriebenen Verfahren zur Oberflächenbehandlung von Polyaryletherketonen (PAEKs);
b) Beschichten der mindestens einen behandelten Teilfläche der Oberfläche des Gegenstandes mit einer Zusammensetzung, die eine chemische Verbindung enthält mit chemischen Gruppen, die eine kovalente Bindung mit den auf der Oberfläche des Gegenstandes ausgebildeten Hydroxyalkyl- und/oder Hydroxyarylgruppen ausbilden können.

Für die Oberflächenbehandlung der mindestens einen Teilfläche der Oberfläche des Gegenstandes, der ein oder mehrere Polyaryletherketone (PAEKs) enthält, ist vorzugweise jeder hierin beschriebene Aspekt und jede hierin beschriebene Ausführungsform des erfindungsgemäßen Verfahrens zur Oberflächenbehandlung von Polyaryletherketonen (PAEKs) anwendbar.

Die Zusammensetzung für die Beschichtung der mindestens einen behandelten Teilfläche der Oberfläche des Gegenstandes enthält vorzugsweise biologisches Material oder Biomoleküle. Bevorzugt sind hierbei natürliche(s), künstliche(s), chemisch modifizierte(s) oder biotechnologisch hergestellte(s) biologisches Material oder Biomoleküle.

"Natürlich" bedeutet in diesem Zusammenhang, dass das biologische Material oder die Biomoleküle aus natürlichen Quellen stammen.

"Künstlich" bedeutet in diesem Zusammenhang, dass das biologische Material oder die Biomoleküle über chemische Verfahren außerhalb natürlicher Quellen hergestellt wurden.

"Chemisch modifiziert" bedeutet, dass biologisches Material oder Biomoleküle durch chemische oder biotechnologische Verfahrensschritte modifiziert worden sind. "Biotechnologisch hergestellt" bedeutet, dass das biologische Material oder die Biomoleküle über biotechnologische Verfahren hergestellt wurden.

"Biologisches Material" ist Material biologischen (d.h. pflanzlichen, pilzlichen oder tierischen) Ursprungs. Dabei kann es sich um einzelne Molekülklassen biologischen Ursprungs oder eine Mischung mehrerer Molekülklassen handeln. Auch pflanzliche, pilzliche oder tierische Zellen oder Gewebe fallen unter die Definition von biologischem Material. Geeignetes biologisches Material oder Biomoleküle sind vorzugsweise ausgewählt aus Proteinen, Oligo- oder Polypeptiden, Aminosäuren, Mono-, Oligo- oder Polysacchariden, Proteoglykanen, Glykoproteinen, Glykosaminoglykanen, Lipiden, Glykolipiden, Nukleotiden, Vitaminen und anderen niedermolekularen Verbindungen (i.e. Verbindungen mit einer Molekülmasse von nicht mehr als 800 g/mol) oder Mischungen daraus.

Die Zusammensetzung enthält vorzugsweise zumindest Proteine oder Proteingemische, bevorzugt Proteine oder Proteingemische der tierischen extrazellulären Matrix, wie beispielsweise Gelatine oder Kollagen.

Für das erfindungsgemäße Verfahren ist es notwendig, dass die Zusammensetzung mindestens eine chemische Verbindung enthält mit chemischen Gruppen, die eine kovalente Bindung mit den auf der Oberfläche des Gegenstandes ausgebildeten Hydroxyalkyl- und/oder Hydroxyarylgruppen ausbilden können.

Derartige chemische Gruppen sind beispielsweise Aminogruppen, Alkoholgruppen, Aldehydgruppen, Carboxylgruppen, Halogenidgruppen und Mischungen daraus.

Diese chemischen Gruppen sind vorzugsweise ausgebildet in den oben ausgeführten biologischen Materialien oder Biomolekülen. Die chemischen Gruppen können dabei bereits bei der natürlichen, chemischen oder biotechnologischen Synthese der biologischen Materialien oder Biomoleküle oder durch chemische Modifikation in diese eingeführt worden sein. Besonders bevorzugt finden sich derartige chemische Gruppen in den Aminosäureseitenketten von Proteinen oder Proteingemischen. Beispielsweise können primäre Amine (z.B. aus Lysinseitenketten) und Hydroxyphenylgruppen (z. B. aus Tyrosinseitenketten) mit den Hydroxyalkyl- und/oder Hydroxyarylgruppen, vorzugsweise den addierten Methylolgruppen, der oberflächenbehandelten Polyaryletherketone (PAEKs) kovalente Bindungen über eine Methylengruppe (-CH₂-) ausbilden.

Somit kann eine kovalente Bindung zwischen den chemischen Verbindungen, vorzugsweise dem biologischen Material oder den Biomolekülen, der Beschichtung und den ein oder mehreren oberflächenbehandelten Polyaryletherketonen (PAEKs) auf der mindestens einen Teilfläche der Oberfläche des Gegenstandes hergestellt werden.

Die chemischen Verbindungen der Beschichtung sind vorzugsweise gewebeverträglich (biokompatibel). Durch eine kovalente Bindung der Beschichtung mit der mindestens einen Teilfläche der Oberfläche des Gegenstandes kann somit die Osseointegration des Gegenstandes bei Verwendung beispielweise als Implantat verbessert werden.

Durch die Beschichtung und kovalente Bindung der Beschichtung an die ein oder mehreren oberflächenbehandelten Polyaryletherketone (PAEKs) des Gegenstandes des erfindungsgemäßen Verfahrens kann auf Beschichtungen der Polyaryletherketone (PAEKs) mit metallischen oder keramischen Materialien wie Ti, TiO₂, Ta, Zeolith oder Hydroxyapatit verzichtet werden.

Vorzugsweise werden alle Oberflächen des Gegenstandes zunächst mit dem oben beschriebenen Verfahren oberflächenbehandelt und dann mit dem nun beschriebenen Verfahren beschichtet.

Die Beschichtung kann mit jeglichem denkbaren Verfahren erfolgen.

Vorzugsweise wird die mindestens eine behandelte Teilfläche der Oberfläche des Gegenstands mit der Zusammensetzung durch Elektrospinnen, Elektrospray, Aerosol-Deposition, Rakeln, Tauchbeschichtung oder Spritzbeschichtung beschichtet.

Besonders bevorzugt ist eine Beschichtung durch Elektrospinnen beispielsweise mit Nanofasern der Zusammensetzung, vorzugsweise Nanofasern von Peptiden oder Proteinen, wie beispielsweise Kollagen oder Gelatine.

Bei Elektrospinnexperimenten wurde dabei festgestellt, dass eine stabilere Beschichtung auf den ein oder mehreren oberflächenbehandelten Polyaryletherketonen (PAEKs) des Gegenstandes erzielt werden konnte, wenn der Gegenstand nicht in direktem Kontakt zu der Kollektorelektrode der Elektrospinnapparatur stand.

Die Schichtdicke der Beschichtung liegt üblicherweise im Bereich von 0,1 bis 500 µm, bevorzugt von 0,5 bis 400 µm, stärker bevorzugt von 1 bis 300 µm.

Beim Elektrospinnen von Nanofasern haben die Nanofasern üblicherweise einen mittleren Durchmesser im Bereich von 10 bis 700 nm, bevorzugt von 25 bis 500 nm, stärker bevorzugt 50 bis 300 nm.

Die Beschichtung erfolgt vorzugsweise im engen zeitlichen Zusammenhang mit der Oberflächenbehandlung der mindestens einen Teilfläche der Oberfläche des Gegenstandes nach dem oben beschriebenen Verfahren zur Oberflächenbehandlung. Hierdurch kann verhindert werden, dass die in dem Verfahren zur Oberflächenbehandlung ausgebildeten Hydroxyalkyl- und/oder Hydroxyarylgruppen, vorzugsweise der addierten Methylolgruppen, ungewollte chemische Reaktionen, beispielsweise mit reaktiven Gruppen in der Umgebung oder mit sich selbst, eingehen und somit für eine kovalente Bindung mit den chemischen Gruppen der Beschichtung nicht zur Verfügung stehen.

Nach Ausbildung der kovalenten Bindung zu den ein oder mehreren oberflächenbehandelten Polyaryletherketonen (PAEKs) kann die Zusammensetzung der Beschichtung zusätzlich vernetzt werden. Hierzu können übrige reaktive chemische Gruppen der Zusammensetzung, die keine kovalente Bindung mit den ein oder mehreren oberflächenbehandelten Polyaryletherketonen (PAEKs) eingegangen sind, mit reaktiven Verbindungen, wie beispielsweise Aldehyden, vorzugsweise Formaldehyd, in Kontakt gebracht werden, was eine Vernetzung der chemischen Gruppen zur Folge hat. Eine weitere geeignete Vernetzung kann über Vernetzungsmittel aus der Gruppe der Carbodiimide, wie beispielsweise 1-Ethyl-3-(3-dimethylaminopropyl)carbodiimid (EDC), in Kombination mit Oxidationsmitteln, wie beispielsweise N-hydroxysuccinimid (NHS) oder N-Hydroxysulfosuccinimid-Natriumsalz (Sulfo-NHS), erfolgen.

GegenstandIn einem weiteren Aspekt betrifft die vorliegende Erfindung einen Gegenstand enthaltend ein oder mehrere Polyaryletherketone (PAEKs) und eine Beschichtung auf mindestens einer Teilfläche der Oberfläche des Gegenstands, wobei an der mindestens einen beschichteten Teilfläche der Oberfläche des Gegenstands der/die Polyaryletherketone (PAEKs) Hydroxyalkyl- und/oder Hydroxyarylgruppen enthält/enthalten; und mindestens ein Teil der Hydroxyalkyl- und/oder Hydroxyarylgruppen des Polyaryletherketons/der Polyaryletherketone (PAEKs) kovalente Bindungen mit chemischen Gruppen von mindestens einer chemischen Verbindung in der Beschichtung ausgebildet hat.

Der Gegenstand ist vorzugsweise herstellbar mit dem Verfahren zur Funktionalisierung von oberflächenbehandelten Polyaryletherketonen (PAEKs).

Die Hydroxyalkyl- und/oder Hydroxyarylgruppen werden vorzugsweise durch das Verfahren zur Oberflächenbehandlung von Polyaryletherketonen (PAEKs) auf der mindestens einen Teilfläche der Oberfläche des Gegenstandes ausgebildet.

Dabei ist vorzugweise jeder hierin beschriebene Aspekt und jede hierin beschriebene Ausführungsform des erfindungsgemäßen Verfahrens zur Oberflächenbehandlung von Polyaryletherketonen (PAEKs) und/oder des erfindungsgemäßen Verfahrens zur Funktionalisierung von oberflächenbehandelten Polyaryletherketonen (PAEKs) anwendbar.

Die Beschichtung ist kovalent mit der mindestens einen Teilfläche der Oberfläche des Gegenstands verbunden. Dies führt zu einer ausreichenden strukturellen Integrität zwischen der mindestens einen Teilfläche der Oberfläche des Gegenstands und der Beschichtung, so dass eine Ablösung der Beschichtung, beispielsweise durch Schrumpfung verhindert wird.

Der Gegenstand ist vorzugsweise zytokompatibel. Diese Eigenschaft wird vorzugsweise durch die Beschichtung erzeugt.

Dabei zeigt sich, dass aufgrund der erhöhten Zytokompatibiliät eine verbesserte Adhäsion von Zellen an das Polyaryletherketonmaterial zu beobachten ist. Hierdurch verbessert sich die Osseointegration von PAEK-Implantaten.

Der Gegenstand ist vorzugsweise ein Medizinprodukt und/oder eine biotechnologische Anwendung, vorzugsweise ein Implantat, eine Gerüststruktur für in vitro Anwendungen und/oder eine Gerüststruktur für Zellkulturanwendungen.

Besonders bevorzugt ist der Gegenstand ein orthopädisches oder spinales Implantat.

### Vorteile der Erfindung

Mit Hilfe der erfindungsgemäßen Verfahren lassen sich Beschichtungsmaterialien auf der Oberfläche von Polyaryletherketon (PAEKs)-haltigen Formkörpern kovalent binden, die unterschiedliche chemische und/oder physikalische Eigenschaften im Vergleich zu Polyaryletherketonen (PAEKs) haben. Beispielsweise, wie hier beschrieben, lassen sich mit den hier beschriebenen erfindungsgemäßen Verfahren zytokompatible Beschichtungen kovalent auf der Oberfläche von Polyaryletherketon (PAEKs)-haltigen Formkörpern etablieren.

Zusätzlich erlauben die erfindungsgemäßen Verfahren die Beschichtung von Polyaryletherketon (PAEKs)-haltigen Formkörpern unter Beibehaltung der strukturellen Eigenschaften der Polyaryletherketone (PAEKs).

Durch eine Beschichtung mit einer zytokompatiblen Beschichtung, wie beispielsweise mit einer protein- oder biopolymerhaltigen Beschichtung, ist eine verbesserte Adhäsion von Zellen an das Polyaryletherketonmaterial zu beobachten. Hierdurch verbessert sich die Osseointegration von PAEK-Implantaten.

Die erfindungsgemäßen Polyaryletherketon (PAEKs)-haltigen Formkörper eignen sich somit besonders für Medizinanwendung und Gerüststrukturen in Zellkultur und Tissue-Engineering-Anwendungen. Besonders geeignet sind die erfindungsgemäßen Polyaryletherketon (PAEKs)-haltigen Formkörper als Implantate, insbesondere als orthopädische oder spinale Implantate.

### Beschreibung der Figur

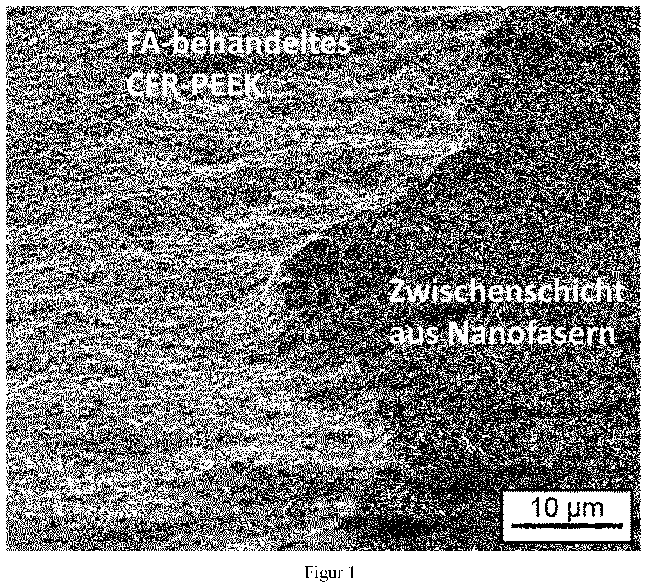

Figur 1 zeigt eine rasterelektronenmikroskopische Aufnahme der Grenzphase eines mit Formaldehyd oberflächenbehandelten Formkörpers aus PEEK (FA-etched PEEK) und einer Beschichtung aus Gelatine, die durch Elektrospinnen von Gelatine-Nanofasern (Intermediate nanofiber layer) auf der Oberfläche des oberflächenbehandelten Formkörpers ausgebildet worden ist. Die Pfeile in der Figur zeigen die Kontaktfläche an der die Fasern der Beschichtung mit der mit Formaldehyd behandelten Oberfläche des Formkörpers verschmelzen durch kovalente Bindung. Durch diese Verschmelzung entsteht eine ausreichende strukturelle Integrität zwischen der Oberfläche des PEEK Formkörpers und der Faserbeschichtung, so dass eine Ablösung der Beschichtung, beispielsweise durch Schrumpfung verhindert wird.

### Beispiele

### Beispiel 1: Aufbringen einer stabilen Beschichtung aus nicht orientierten Gelatine-Nanofasern auf CFR-PEEK-Proben

Für die Beschichtungsexperimente wurde kohlenstofffaserverstärktes Polyetheretherketon (CFR-PEEK) mit 30 % Kohlenstofffasern von POLYTRON Kunststofftechnik (Victrex^{®} PEEK 150CA30, Bergisch Gladbach, Deutschland) verwendet. Es wurden Prüfkörper mit einer Oberfläche von 484 mm² und einer Dicke von 1 mm aus dem Material hergestellt. Die CFR-PEEK Prüfkörper wurden mit ddH₂O gereinigt und anschließend bei Raumtemperatur in eine 37%ige Formaldehydlösung (Carl Roth, Deutschland) getaucht. Nach 30 Minuten wurden die Proben aus der Formaldehydlösung entfernt und die überschüssige Flüssigkeit mit einem Papiertuch entfernt. Die Formkörper wurden direkt nach der Formaldehydaktivierung mit Protein-Nanofasern beschichtet.

Für die Nanofaser-Beschichtung wurden die Formaldehyd-aktivierten CFR-PEEK-Formkörper auf einen Plattenkollektor in einem speziell angefertigten Versuchsaufbau für Elektrospinnen gelegt. 20% (w/v) Gelatine wurde in 50% (v/v) Essigsäure aufgelöst und in eine 20 mL Spritze (B. Braun Perfusor) überführt. Der Auslass der Spritze wurde mit einer stumpfen 21G Kanüle über einen Infusionsschlauch (B. Braun Perfusor) verbunden. Die Spritze wurde in einer Spritzenpumpe platziert (neMESYS, Cetoni GmbH, Deutschland) mit einer softwaregesteuerten Vorschubgeschwindigkeit. Die Spritze wurde an eine Hochspannungs-Gleichstromquelle (Heinzinger, Deutschland) angeschlossen und über einer geerdeten Kupferplatte mit einer Fläche von 10x10 cm² im einem vertikalen Abstand von 12 cm platziert. Die Spannung wurde auf 12 kV eingestellt und die Einspeisungsgeschwindigkeit auf 5 µL/min festgelegt. Auf jeden Formaldehyd aktivierten CFR-PEEK-Formkörper wurden 0,25 mL Gelatinelösung durch Elektrospinnen abgeschieden.

Die mit Nanofasern beschichteten CFR-PEEK-Probekörper wurden anschließend 24 Stunden lang bei 37 °C getrocknet. Danach wurden die Probekörper in einen Exsikkator (Gesamtvolumen ca. 2,4 L) über einem Reservoir von 37 % Formaldehydlösung in Wasser gelegt. Für je 30 mg Gelatine (Trockenmasse) wurden 10 mL Formaldehydlösung verwendet.

Die Proben wurden 105 Minuten lang im Exsikkator inkubiert, um die über Elektrospinnen aufgetragene Gelatinefaserbeschichtung zu stabilisieren.

Dieses Verfahren führt zu einer Zwischenschicht aus Fasern, die fest an der CFR-PEEK-Oberfläche haften. Um diese Schicht vollständig zu entfernen, muss sie abgeschabt oder abgeschliffen werden. Die Fasern, die auf dieser Zwischenschicht abgeschieden wurden sind wirksam vernetzt und bilden eine stabile Beschichtung mit einer durchschnittlichen Dicke von 211 ± 49 µm und einer Trockenmasse von 3,1 ± 0,3 mg. Der mittlere Durchmesser der abgeschiedenen Fasern wurde bestimmt durch Rasterelektronenmikroskopie und Bildanalyse auf 143 ± 29 nm für unbehandelte Fasern und einem leichten Anstieg auf 155 ± 34 nm nach der Formaldehyd-Vernetzung.

Dieses Verfahren führt zu einer weniger effizienten Beschichtung von PEEK (ohne Zusatzmaterial wie Glasfaser, Kohlenstofffasern oder Ähnliches), da die Homogenität des elektrischen Felds durch den direkten Kontakt zur Kollektorelektrode beeinträchtigt wurde, wodurch die isolierenden Eigenschaften von PEEK verstärkt wurden. Die Effizienz der PEEK-Beschichtung ließ sich durch Änderung des Elektrospinnanlage wie in Beispiel 5 beschrieben signifikant verbessern.

### Beispiel 2: Aufbringen einer stabilen Beschichtung aus orientierten Gelatine-Nanofasern auf CFR-PEEK-Probekörper

CFR-PEEK-Probekörper wurden wie in Beispiel 1 beschrieben hergestellt und voraktiviert. Nach der Formaldehyd-Vorbehandlung wurden die Probekörper in einer Elektrospinnanlage zwischen zwei geerdeten Kupfer-Kollektorplatten mit einer Fläche von je 2x1cm² gelegt. Anschließend wurden die Probekörper mit Gelatine-Nanofasern durch Elektrospinnen wie in Beispiel 1 beschrieben beschichtet. Der geänderte Kollektoraufbau führte zu einer Beschichtung mit ausgerichteten Fasern.

### Beispiel 3: Verbesserte Zytokompatibilität und Zellproliferation

CFR-PEEK-Probekörper wurden auf die Maße 10x10x1 mm³ zugeschnitten und anschließend mit einem Schleifpapier mit 500er Körnung geschliffen, um eine Oberflächenrauheit von 0,37 µm zu erreichen. Die Probekörper wurden voraktiviert durch Inkubation in 37%iger Formaldehydlösung wie in Beispiel 1 beschrieben. Die Proben wurden dann mit Gelatine-Nanofasern durch Elektrospinnen beschichtet wie in Beispiel 1 beschrieben. Für dieses Verfahren wurden 0,1 mL Gelatinelösung elektrogesponnen und auf sechs Probekörper aufgebracht. Nach dem Elektrospinnen wurden die Probekörper 24 Stunden lang bei 37 °C getrocknet, bevor die Fasern durch Formaldehyd-Begasung vernetzt wurden, wie in Beispiel 1 beschrieben. Um die Probekörper für Zellkulturexperimente zu optimieren, wurden die Probekörper bei 50 °C und 80 mbar für 48 h gelagert und in eine 24-Loch-Platte gegeben. Jeder Probekörper wurde desinfiziert, indem er für 2 h bei Raumtemperatur unter einer biologischen Werkbank in 1 mL 70%igem Ethanol getaucht wurde. Das Ethanol wurde mit steriler phosphatgepufferter Kochsalzlösung (PBS) von den Probekörpern gespült. Die Probekörper wurden vor der Zellaussaat in 1 mL Delbecco's Modified Eagle Medium F-12 (DMEM/F-12) bei 37 °C für 24 h gelagert.

Humane Chondrosarkomzellen (SW1353) wurden in einem Kulturmedium aus DMEM/F-12, 10 % fötalem Rinderserum (FBS), 1% L-Glutamin und 1% Penicillin/Streptomycin bei 37 °C in feuchter Atmosphäre bei 5 % CO₂ bis zur Konfluenz kultiviert. Die Zellen wurden in Polystyrol-Loch-Platten (Kontrolle), auf unbehandelten und auf gelatinebeschichteten CFR-PEEK-Proben (n=6) mit einer Dichte von 5000 Zellen/cm² ausgesät. Zellviabilitätsassays (CellTiter-Blue, Promega) wurden jeweils an den Tagen 0, 1, 2, 3, 4, 7 und 8 durchgeführt. Zellvitalität in den Kontrollgruppen lag bei ≥97,8%. Die Zellzahl auf den gelatinebeschichteten CFR-PEEK Probekörper war nach 192 Stunden ca. 33% höher im Vergleich zu der Zellzahl auf den unbehandelte CFR-PEEK Probekörpern. Im Vergleich zu Beispiel 1 führte das hier beschriebene modifizierte Beschichtungsverfahren zu einem starken Anstieg der Zytokompatibilität.

### Beispiel 4: Alternative Vernetzungsstrategie

CFR-PEEK-Probekörper (22x22x1 mm³) wurden wie in Beispiel 1 beschrieben hergestellt, gereinigt und 30 Minuten lang bei Raumtemperatur in 37%ige Formaldehydlösung getaucht. Die Probekörper wurden anschließend aus der Flüssigkeit entfernt. Die Probekörper wurden anschließend getrocknet und mit Gelatinefasern durch Elektrospinnen wie in Beispiel 1 beschrieben beschichtet. Nach dem Elektrospinnen wurden die faserbeschichteten CFR-PEEK-Proben 24 Stunden lang bei 37 °C getrocknet. Die Stabilisierung der Fasern erfolgte durch chemische Quervernetzung mit 1-Ethyl-3-(3-dimethylaminopropyl)carbodiimid-Hydrochlorid (EDC-HCl, Merck, Deutschland). 100 mM EDC-HCl wurde in Isopropanol gelöst. 3 mL EDC-Lösung pro mg Gelatinefasern (Trockengewicht) wurden in einen kleinen Behälter gegeben. Die mit Fasern beschichteten CFR-PEEK- Probekörper wurden anschließend in dem Behälter für 2 Stunden bei Raumtemperatur in die EDC-Lösung getaucht.

Der mittlere Faserdurchmesser der abgeschiedenen und EDC-vernetzten Fasern betrug 450 ± 34 nm (bestimmt durch Rasterelektronenmikroskopie und Bildanalyse). Es bildete sich eine Zwischenschicht, die stark an der CFR-PEEK-Oberfläche anhaftete und zur vollständigen Entfernung abgekratzt oder abgeschliffen werden musste. Allerdings war die Vernetzung der auf dieser Zwischenschicht aufgebrachten Fasern weniger effektiv als in Beispiel 1.

### Beispiel 5: Anwendung von Geräten im Produktionsmaßstab

PEEK- und CFR-PEEK-Probekörper wurden wie in Beispiel 1 beschrieben hergestellt und in ein Nanospider NS 1WS 500 U Elektrospinngerät (Elmarco, Tschechische Republik) eingefügt. Hierfür wurden die Probekörper mit doppelseitigem Klebeband auf dem Polypropylensubstrat fixiert. Eine Protein/Polymer-Lösung, die natives Kollagen, Poly(ethylenoxid) und Hydroxyapatit enthielt (SpinPlant GmbH, Deutschland), wurde bei 80 kV 30 Minuten lang elektrogesponnen und auf die CFR-PEEK-Probekörper aufgebracht. Da die Probekörper nicht in direktem Kontakt mit der Kollektorelektrode standen, wurden die Nanofasern gleich effizient auf PEEK- und CFR-PEEK-Proben abgeschieden.

### Beispiel 6: 3D-Beschichtung von CFR-PEEK-Implantaten

Ein Block aus CFR-PEEK wurde hergestellt und durch Eintauchen in eine 37%ige Formaldehydlösung voraktiviert wie in Beispiel 1 beschrieben. Der Block wurde anschließend auf eine speziell angefertigte Drehvorrichtung montiert und diese in der in Beispiel 1 beschriebenen Elektrospinnanlage zwischen der geerdeten Kupfer-Kollektorplatte und der an die Hochspannungs-Gleichstromquelle angeschlossenen Nadel platziert. Das Elektrospinnen wurde wie in Beispiel 1 beschrieben durchgeführt. Dabei wurde die Drehvorrichtung mit dem CFR-PEEK-Block mit einer Geschwindigkeit von ca. 10 U/min gedreht. Dies führte zu einer homogenen Beschichtung aller Seiten des CFR-PEEK-Blocks. Anschließende Vernetzung durch Formaldehyd-Begasung wie in Beispiel 1 beschrieben ergab eine stabile Beschichtung.

## Patentansprüche

1. Verfahren zur Oberflächenbehandlung von Polyaryletherketonen (PAEKs) umfassend folgende Schritte:
• Bereitstellen eines Gegenstandes, der ein oder mehrere Polyaryletherketone (PAEKs) enthält;
• in Kontakt bringen von mindestens einer Teilfläche der Oberfläche des Gegenstandes, der Polyaryletherketon (PAEK) enthält, mit einem Aldehyd,
wobei das Aldehyd mit dem/den Polyaryletherketon(en) (PAEKs) an der mindestens einen Teilfläche der Oberfläche des Gegenstands unter Ausbildung einer Hydroxyalkyl- und/oder Hydroxyarylgruppe reagiert.

2. Das Verfahren gemäß Anspruch 1, wobei die Polyaryletherketone (PAEKs) ausgewählt sind aus Polyetherketonen (PEK), Polyetheretherketonen (PEEK), Polyetherketonketonen (PEKK), Polyetheretheretherketonen (PEEEK), Polyetheretherketonketonen (PEEKK), Polyetherketonetherketonketonen (PEKEKK) oder Mischungen daraus.

3. Das Verfahren gemäß Anspruch 1 oder 2, wobei der Gegenstand, der ein oder mehrere Polyaryletherketone (PAEKs) enthält, eine Mischung aus dem/der Polyaryletherketone (PAEKs) mit einem anorganischen Strukturmaterial, vorzugsweise mit Glasfasern, Kohlenstofffasern und/oder Hydroxyapatit, enthält.

4. Das Verfahren gemäß einem der vorherigen Ansprüche, wobei das Aldehyd ausgewählt ist aus Formaldehyd (Methanal), Glyoxal (Ethandial), Succinaldehyd (Butandial) und Glutaraldehyd (Pentandial) oder Mischungen daraus.

5. Das Verfahren gemäß einem der vorherigen Ansprüche, wobei das Aldehyd als Flüssigkeit, als Gas oder als Aerosol mit mindestens einer Teilfläche der Oberfläche des Gegenstandes, der ein oder mehrere Polyaryletherketone (PAEKs) enthält, in Kontakt gebracht wird.

6. Das Verfahren gemäß einem der vorherigen Ansprüche, wobei die mindestens eine Teilfläche der Oberfläche des Gegenstandes, der ein oder mehrere Polyaryletherketone (PAEKs) enthält, zusätzlich mindestens einer weiteren Oberflächenbehandlung, wie beispielsweise einer Plasmabehandlung, unterzogen wird.

7. Verfahren zur Funktionalisierung von oberflächenbehandelten Polyaryletherketonen (PAEKs), umfassend folgende Schritte:
a) Behandeln von mindestens einer Teilfläche der Oberfläche eines Gegenstandes, der ein oder mehrere Polyaryletherketone (PAEKs) enthält, mit dem Verfahren gemäß einem der vorherigen Ansprüche;
b) Beschichten der mindestens einen behandelten Teilfläche der Oberfläche des Gegenstandes mit einer Zusammensetzung, die eine chemische Verbindung enthält mit chemischen Gruppen, die eine kovalente Bindung mit den auf der Oberfläche des Gegenstandes ausgebildeten Hydroxyalkyl- und/oder Hydroxyarylgruppen ausbilden können.

8. Das Verfahren gemäß Anspruch 7, wobei die Zusammensetzung biologisches Material oder Biomoleküle enthält, vorzugsweise ausgewählt aus natürlichen, künstlichen, chemisch modifizierten oder biotechnologisch hergestelltem biologischen Material oder Biomolekülen, wie Proteinen, Oligo- oder Polypeptiden, Aminosäuren, Mono-, Oligo- oder Polysacchariden, Proteoglykanen, Glykoproteinen oder Glykosaminoglykanen, Lipiden, Glykolipiden, Nukleotiden, Vitaminen und anderen niedermolekulare Verbindungen oder Mischungen daraus, besonders bevorzugt Kollagen oder Gelatine.

9. Das Verfahren gemäß Anspruch 7 oder 8, wobei die chemischen Gruppen, die eine kovalente Bindung mit den auf der Oberfläche des Gegenstandes ausgebildeten Hydroxyalkyl- und/oder Hydroxyarylgruppen ausbilden können, ausgewählt sind aus Aminogruppen, Alkoholgruppen, Aldehydgruppen, Carboxylgruppen, Halogenidgruppen und Mischungen daraus.

10. Das Verfahren gemäß einem der Ansprüche 7 bis 9, wobei die mindestens eine behandelte Teilfläche der Oberfläche mit der Zusammensetzung durch Elektrospinnen, Elektrospray, Aerosol-Deposition, Rakeln, Tauchbeschichtung oder Spritzbeschichtung beschichtet wird.

11. Das Verfahren gemäß einem der Ansprüche 7 bis 10, wobei nach der Beschichtung die Zusammensetzung vernetzt wird.

12. Ein Gegenstand enthaltend ein oder mehrere Polyaryletherketone (PAEKs) und eine Beschichtung auf mindestens einer Teilfläche Oberfläche des Gegenstands, wobei
an der mindestens einen beschichteten Teilfläche der Oberfläche des Gegenstands der/die Polyaryletherketone (PAEKs) Hydroxyalkyl- und/oder Hydroxyarylgruppen enthält/enthalten; und
mindestens ein Teil der Hydroxyalkyl- und/oder Hydroxyarylgruppen des Polyaryletherketons/der Polyaryletherketone (PAEKs) kovalente Bindungen mit chemischen Gruppen von mindestens einer chemischen Verbindung in der Beschichtung ausgebildet hat.

13. Der Gegenstand gemäß Anspruch 12, hergestellt nach dem Verfahren gemäß einem der Ansprüche 7 bis 11.

14. Der Gegenstand gemäß Anspruch 12 oder 13, wobei der Gegenstand zytokompatibel ist.

15. Der Gegenstand gemäß einem der Ansprüche 12 bis 14, wobei der Gegenstand ein Medizinprodukt und/oder eine biotechnologische Anwendung, vorzugsweise ein Implantat, eine Gerüststruktur für in vitro Anwendungen und/oder eine Gerüststruktur für Zellkulturanwendungen ist.

## Claims

1. A method for surface treatment of polyaryletherketones (PAEKs), comprising the following steps:
• Providing an object that contains one or more polyaryletherketones (PAEKs);
• Bringing at least one partial surface of the surface of the object that contains polyaryletherketone (PAEK) into contact with an aldehyde,
wherein the aldehyde reacts with the one or more polyaryletherketones (PAEKs) on the at least one partial surface of the surface of the object, forming a hydroxyalkyl- and/or hydroxyaryl group.

2. The method according to Claim 1, wherein the polyaryletherketones (PAEKs) are selected from polyetherketones (PEK), polyether ether ketones (PEEK), polyether ketone ketones (PEKK), polyether ether ether ketones (PEEEK), polyether ether ketone ketones (PEEKK), polyether ketone ether ketone ketones (PEKEKK) or mixtures thereof.

3. The method according to Claim 1 or 2, wherein the object that contains one or more polyaryletherketones (PAEKs) contains a mixture of the one or more polyaryletherketones (PAEKs) with an inorganic structural material, preferably with glass fibres, carbon fibres and/or hydroxyapatite.

4. The method according to any one of the preceding claims, wherein the aldehyde is selected from formaldehyde (methanal), glyoxal (ethanedial), succinaldehyde (butanedial) and glutaraldehyde (pentanedial) or mixtures thereof.

5. The method according to any one of the preceding claims, wherein the aldehyde in the form of a liquid, a gas or an aerosol is brought into contact with at least one partial surface of the surface of the object that contains one or more polyaryletherketones (PAEKs).

6. The method according to any one of the preceding claims, wherein the at least one partial surface of the surface of the object that contains one or more polyaryletherketones (PAEKs) additionally undergoes at least one further surface treatment process, such as a plasma treatment, for example.

7. A method for functionalisation of surface-treated polyaryletherketones (PAEKs), comprising the following steps:
a) Treating at least one partial surface of the surface of an object that contains one or more polyaryletherketones (PAEKs) with the method according to one of the preceding claims;
b) Coating the at least one treated partial surface of the surface of the object with a composition that contains a chemical compound with chemical groups that are able to form a covalent bond with hydroxyalkyl- and/or hydroxyaryl groups formed on the surface of the object.

8. The method according to Claim 7, wherein the composition contains biological material or biomolecules, preferably selected from natural, synthetic, chemically modified or biotechnologically manufactured biological material or biomolecules such as proteins, oligo- or polypeptides, amino acids, mono-, oligo- or polysaccharides, proteoglycans, glycoproteins or glycosaminoglycans, lipids, glycolipids, nucleotides, vitamins and other low molecular weight compounds or mixtures thereof, particularly preferably collagen or gelatine.

9. The method according to Claim 7 or 8, wherein the chemical groups that are able to form a covalent bond with the hydroxyalkyl- and/or hydroxyaryl groups formed on the surface of the object are selected from amino groups, alcohol groups, aldehyde groups, carboxyl groups, halide groups, and mixtures thereof.

10. The method according to any one of Claims 7 to 9, wherein the at least one treated partial surface of the surface is coated with the composition by electrospinning, electrospraying, aerosol deposition, doctor blade application, dip coating or spray coating.

11. The method according to any one of Claims 7 to 10, wherein after the coating the composition is crosslinked.

12. An object containing one or more polyaryletherketones (PAEKs) and a coating on at least one partial surface of a surface of the object, wherein the polyaryletherketone(s) (PAEKs) on the at least one coated partial surface of the surface of the object contain/contains hydroxyalkyl- and/or hydroxyaryl groups; and at least some of the hydroxyalkyl- and/or hydroxyaryl groups of the polyaryletherketone/polyaryletherketones (PAEKs) has formed covalent bonds with chemical groups of at least one chemical compound in the coating.

13. The object according to Claim 12, produced with the method according to any one of Claims 7 to 11.

14. The object according to Claim 12 or 13, wherein the object is cytocompatible.

15. The object according to any one of Claims 12 to 14, wherein the object is a medicinal product and/or a biotechnological application, preferably an implant, a framework structure for *in vitro* applications, and/or a framework structure for cell culture applications.

## Revendications

1. Procédé de traitement de surface de polyaryléthercétones (PAEK), comprenant les étapes suivantes :
• préparation d'un article, qui contient un ou plusieurs polyaryléthercétones (PAEK) ;
• mise en contact d'au moins une partie de la surface de l'article qui contient le polyaryléthercétone (PAEK) avec un aldéhyde, dans lequel l'aldéhyde réagit avec le ou les polyaryléthercétones (PAEK) présents sur au moins une partie de la surface de l'article pour former un groupe hydroxyalkyle et/ou hydroxyaryle.

2. Procédé selon la revendication 1, dans lequel les polyaryléthercétones (PAEK) sont choisies parmi les polyéthercétones (PEK), les polyétheréthercétones (PEEK), les polyéthercétonecétones (PEKK), les polyétheréthercétones (PEEEK), les polyétheréthercétonecétones (PEEKK), les polyéthercétoneéthercétonecétones (PEKEKK), ou leurs mélanges.

3. Procédé selon la revendication 1 ou 2, dans lequel l'article qui contient une ou plusieurs polyaryléthercétones (PAEK) contient un mélange de polyaryléthercétones (PAEK) avec un matériau structurel inorganique, de préférence des fibres de verre, des fibres de carbone et/ou de l'hydroxyapatite.

4. Procédé selon une quelconque des revendications précédentes, dans lequel l'aldéhyde est choisi parmi le formaldéhyde (méthanal), le glyoxal (éthanedial), le succinaldéhyde (butanédial) et le glutaraldéhyde (pentanédial), ou leurs mélanges.

5. Procédé selon une quelconque des revendications précédentes, dans lequel l'aldéhyde est mis en contact, sous forme liquide, gazeuse ou aérosol, avec au moins une partie de la surface de l'article qui contient une ou plusieurs polyaryléthercétones (PAEK).

6. Procédé selon une quelconque des revendications précédentes, dans lequel la au moins une zone partielle de la surface de l'article, qui contient une ou plusieurs polyaryléthercétones (PAEK) est en outre soumise à au moins un traitement de surface supplémentaire, comme par exemple un traitement plasma.

7. Procédé de fonctionnalisation de polyaryléthercétones (PAEK) traitées en surface, comprenant les étapes suivantes :
a) traitement d'au moins une surface partielle d'un article qui contient une ou plusieurs polyaryléthercétones (PAEK) par le procédé selon une quelconque des revendications précédentes ;
b) revêtement de la ou des surfaces partielles traitées de l'article avec une composition qui contient un composé chimique possédant des groupes chimiques capables de former une liaison covalente avec les groupes hydroxyalkyle et/ou hydroxyaryle formés à la surface de l'article.

8. Procédé selon la revendication 7, dans lequel la composition contient des matières biologiques ou des biomolécules, de préférence choisies parmi des matières biologiques ou des biomolécules naturelles, artificielles, chimiquement modifiées ou produites par biotechnologie, telles que des protéines, des oligo- ou polypeptides, des acides aminés, des mono-, oligo- ou polysaccharides, des protéoglycanes, des glycoprotéines ou des glycosaminoglycanes, des lipides, des glycolipides, des nucléotides, des vitamines et d'autres composés de faible poids moléculaire ou leurs mélanges, de préférence le collagène ou la gélatine.

9. Procédé selon la revendication 7 ou 8, dans lequel les groupes chimiques capables de former une liaison covalente avec les groupes hydroxyalkyle et/ou hydroxyaryle formés à la surface de l'article sont choisis parmi les groupes amino, les groupes alcool, les groupes aldéhyde, les groupes carboxyle, les groupes halogénure et leurs mélanges.

10. Procédé selon une quelconque des revendications 7 à 9, dans lequel la ou les zones de surface partielles traitées sont revêtues de la composition par électrofilage, électrospray, dépôt par aérosol, revêtement à la lame, revêtement par immersion ou revêtement par pulvérisation.

11. Procédé selon une quelconque des revendications 7 à 10, dans lequel, après revêtement, la composition est réticulée.

12. Article contenant une ou plusieurs polyaryléthercétones (PAEK) et un revêtement sur au moins une zone de surface partielle de l'article, dans lequel le/les polyaryléthercétones (PAEK) contient/contiennent des groupes hydroxyalkyle et/ou hydroxyaryle sur la ou les zones de surface partielle revêtues de l'article ; et
au moins certains des groupes hydroxyalkyle et/ou hydroxyaryle des polyaryléthercétones (PAEK) ont formé des liaisons covalentes avec des groupes chimiques d'au moins un composé chimique dans le revêtement.

13. Article selon la revendication 12, produit par le procédé selon une quelconque des revendications 7 à 11.

14. Article selon la revendication 12 ou 13, **caractérisé en ce qu'**il est cytocompatible.

15. Article selon une quelconque des revendications 12 à 14, **caractérisé en ce qu'**il est un dispositif médical et/ou une application biotechnologique, de préférence un implant, une structure d'échafaudage pour des applications in vitro et/ou une structure d'échafaudage pour des applications de culture cellulaire.
